# EUROPÄISCHE PATENTSCHRIFT

(11) **EP 3 337 400 B1**
(45) Veröffentlichungstag und Bekanntmachung des Hinweises auf die Patenterteilung: **27.01.2021**
(21) Anmeldenummer: 16753359.5
(22) Anmeldetag: 16.08.2016
(51) Int. Cl.: A61B 5/11

(54) **ERFASSUNGSVORRICHTUNG ZUM ERFASSEN UND ÜBERWACHEN EINER KÖRPERHALTUNG ODER EINES BEWEGUNGSABLAUFES EINES KÖRPERTEILS**
DETECTION DEVICE FOR DETECTING AND MONITORING A BODY POSTURE OR A SEQUENCE OF MOVEMENTS OF A BODY PART
DISPOSITIF DE DÉTECTION DESTINÉ À LA DÉTECTION ET À LA SURVEILLANCE DU MAINTIEN POSTURAL OU DU DÉROULEMENT D'UN MOUVEMENT D'UNE PARTIE DU CORPS

(30) Priorität: 20.08.2015 DE 102015113816
(43) Veröffentlichungstag der Anmeldung: 27.06.2018
(73) Patentinhaber: VIBRA - TAPE GmbH & Co. KG, 70736 Fellbach (DE)
(72) Erfinder: LACKER, Michael, 71332 Waiblingen (DE)
(74) Vertreter: Mammel und Maser
(86) Internationale Anmeldenummer: PCT/EP2016/069449
(87) Internationale Veröffentlichungsnummer: WO 2017/029297

(56) Entgegenhaltungen:
- WO-A1-2009/074928
- WO-A1-2014/128090
- WO-A1-2014/138204
- DE-A1-102008 052 406
- US-A1- 2015 101 417
- D.-H. KIM ET AL: "Epidermal Electronics", SCIENCE, Bd. 333, Nr. 6044, 12. August 2011 (2011-08-12), Seiten 838-843, XP055298216, US ISSN: 0036-8075, DOI: 10.1126/science.1206157
- RAKIBET OSMAN O ET AL: "Epidermal Passive RFID Strain Sensor for Assisted Technologies", IEEE ANTENNAS AND WIRELESS PROPAGATION LETTERS, Bd. 13, 30. April 2014 (2014-04-30), Seiten 814-817, XP011546816, ISSN: 1536-1225, DOI: 10.1109/LAWP.2014.2318996 [gefunden am 2014-04-30]

## Beschreibung

Die Erfindung betrifft eine Erfassungsvorrichtung zum Erfassen und Überwachen einer Körperhaltung oder eines Bewegungsablaufes eines Körperteils sowie ein Verfahren zur Erkennung und Überwachung hierzu.

Aus der DE 20 2012 005 018 U1 ist ein Überwachungs-, Korrektur- und Konditionierungsgerät für eine Körperhaltung einer Person bekannt. Dieses System umfasst eine oder mehrere Kameras sowie eine Datenverarbeitungs-Software. Diese umfasst eine Haltungserkennungs-Software. Die mittels der Kamera von der zu überwachenden Person erfassten Bilder werden mit dieser Haltungserkennungs-Software ausgewertet, um zu überprüfen, ob die optimale ergonomische Haltung vorliegt oder während der Arbeit an einem Schreibarbeits- oder Bildschirm-Arbeitsplatz aufrechterhalten bleibt. Bei Abweichungen der optimalen ergonomischen Haltung gibt das Programm Korrekturanweisungen und Tipps zur Vorbeugung und Eigenbehandlung.

Dieses Überwachungs-, Korrektur- und Konditionierungsgerät für Körperhaltungen ist ausschließlich an den Einsatz an einem Arbeitsplatz gebunden und technologisch sehr aufwändig.

Aus der DE 20 2005 015 889 U1 ist eine Vorrichtung zur Überwachung der Haltung und/oder Bewegung eines menschlichen Körperteils bekannt. Dabei ist vorgesehen, dass die Vorrichtung zur Überwachung der Haltung und/oder Bewegung eines menschlichen Körpers zumindest einen Sensor aufweist, der am überwachenden Körperteil angebracht wird. Beispielsweise kann dieser Sensor, der als Dehnmessstreifen ausgebildet sein kann, an einer Bandage befestigt werden oder einem entsprechenden Bekleidungsstück, um dieses am Körper zu positionieren.

Aus der DE 10 2008 052 406 A1 ist des Weiteren eine Vorrichtung zum Erfassen von Parametern zur Charakterisierung von Bewegungsabläufen am menschlichen Körper bekannt, welche einen Biegesensor umfasst. Dieser Befestigungssensor umfasst ein Befestigungspflaster zum Befestigen des Biegesensors auf der Haut des menschlichen Körpers. Dieses Befestigungselement umfasst einen Hohlraum, in welchen ein biegesensitiver Detektor eingesetzt wird. Dieser Detektor 10 besteht aus einem band- oder streifenförmigen Substrat, welches aus Federstahl ausgebildet ist. Auf einander gegenüberliegenden Seiten des Substrats sind jeweils entgegengesetzt zueinander ausgerichtet Dehnungsstreifen angeordnet. Diese Vielzahl der Dehnungsstreifen erfasst eine mechanische Formung des Substrates.

Aus der DE 10 2008 052 406 A1 ist ein Biegesensor zur Erfassung von Funktionsparametern zur Charakterisierung von Bewegungsabläufen am menschlichen Körper bekannt. Dieser Bewegungssensor umfasst ein Gehäuse, das unmittelbar am Körper befestigt ist. In dem Gehäuse sind mehrere Dehnmessstreifen in Reihe zueinander und in gleicher Ausrichtung vorgesehen. Von dem Gehäuse führen elektrische Leitungen zu einer getrennt dazu angeordneten Datenverarbeitungseinrichtung, die auch am Körper getragen wird.

Aus der WO 2014/128090 A1 ist eine Vorrichtung zur Überwachung einer regelmäßigen Atmung an einem menschlichen Körper bekannt. Diese Vorrichtung umfasst ein flexibles Substrat, welches auf die Haut aufgeklebt wird. Dieses flexible Substrat ist über Leitungen mit einem Controller verbunden, der in einem Plastikgehäuse angeordnet ist. Innerhalb diesem Plastikgehäuse ist eine Leiterplatte vorgesehen. Diese Leiterplatte wird mit einer Batterie betrieben. Die Leiterplatte ist mit LEDs bestückt und einer Alarmvorrichtung verbunden, die in dem Plastikgehäuse angeordnet ist.

Aus der D.-H. KIM ET AL: "Epidermal Electronic", science, vol. 333, no. 6044, 12. August 2011, ISSN: 0036-8075, DOI: 10.1126/science.1206157 ist eine Elektronik bekannt, welche unmittelbar auf die Haut aufgeklebt wird. Diese Elektronik wird auf einem wasserlöslichen Film bereitgestellt. Dieser löst sich nach dem Aufbringen der Elektronik die Haut auf.

Aus der Veröffentlichung "Epidermal passive RFID strain sensor for assisted technology, IEEE antennas and wireless propagation letters, vol. 13, 30. April 2014, XP011546816, ISSN: 1536-125, DOI: 10.1109/LAWP.2014.2318996 ist ein passiver und drahtloser Dehnungssensor für eine Haut bekannt, der ein RFID-Element umfasst.

Der Erfindung liegt die Aufgabe zugrunde, eine Erfassungsvorrichtung zum Erfassen und Überwachen einer Körperhaltung oder eines Bewegungsablaufs eines Körperteils, insbesondere die Einnahme einer vorgegebenen Position oder die Durchführung eines Bewegungs- oder Trainingsablaufs des Körperteils, vorzuschlagen, welche eine Benutzung unabhängig und nicht ortsgebunden, insbesondere nicht an einen Arbeitsplatz gebunden, ermöglicht.

Diese Aufgabe wird durch eine Erfassungsvorrichtung nach Anspruch 1 gelöst. Eine solche Erfassungseinrichtung ermöglicht, dass diese in einfacher Weise unmittelbar auf der Haut des Benutzers anbringbar ist und somit bereits geringe Veränderungen in der Körperhaltung oder eines Bewegungsablaufes erfasst werden können. Zudem weist diese Erfassungsvorrichtung den Vorteil auf, dass diese unter herkömmlicher Kleidung getragen werden kann, ohne dass dies von außen sichtbar ist oder auffällig wäre. Des Weiteren kann eine solche Erfassungsvorrichtung an verschiedenen Körperregionen eingesetzt werden. Zudem ist der Benutzer ortsungebunden in der Anwendung einer solchen Erfassungsvorrichtung.

Das Trägermaterial mit der Dehnmesseinrichtung und der Schaltung ist als ein Einwegprodukt ausgebildet. Eine solche Erfassungseinrichtung ist für den einmaligen Gebrauch bestimmt. Diese Erfassungsvorrichtung wird an den betreffenden Körperstellen des Benutzers aufgeklebt. Nachdem der gewünschte Überwachungszeitraum abgelaufen ist oder eine Klebewirkung des Klebematerials auf der Trägervorrichtung seine Wirkung verliert, wird diese Erfassungsvorrichtung entsorgt. Darauffolgend ist bei einer erneuten Überwachung die Verwendung einer neuen Erfassungsvorrichtung vorgesehen.

Des Weiteren ist das Trägerelement tapeförmig ausgebildet. Vorzugsweise ist ein längliches streifenförmiges Material zur Herstellung des Trägerelementes vorgesehen, welches vorzugsweise in unterschiedlichen Längen und/oder Breiten gebrauchsfertig bereitgestellt wird. In Abhängigkeit des Anwendungsfalles können lange streifenförmige Erfassungsvorrichtungen beispielsweise entlang der Wirbelsäule eingesetzt werden. Des Weiteren können kurze streifenförmige Trägerelemente beispielsweise im Hals- oder Nackenbereich eingesetzt werden.

Bevorzugt ist das Trägerelement als ein Gewebeband, eine atmungsaktive und/oder luftdurchlässige Folie, als Vlies oder Filz ausgebildet. Somit ist die Erfassungsvorrichtung flexibel in der Anpassung an verschiedene Körperkonturen und Körperbereiche. Bevorzugt werden sogenannte Tapes eingesetzt, wie diese aus der Kinesiologie bekannt sind.

Das Trägerelement nimmt ein Speicherelement und einen Beschleunigungssensor auf. Dieses Speicherelement kann in die Schaltung integriert sein.

Die Erfassungsvorrichtung sieht vor, dass die Dehnmesseinrichtung und die Schaltung und das Speicherelement und der Beschleunigungssensor auf dem Trägerelement aufgebracht sind. Beispielsweise können diese Komponenten auf dem Trägerelement aufgeklebt, aufgedruckt, aufgerakelt, aufgegossen und/oder auch aufgesprüht werden. Dadurch ist ein Aufbau mit einer geringen Schichtdicke und einer hohen Flexibilität zur Anpassung an Körperkonturen ermöglicht.

Die Dehnmesseinrichtung und die Schaltung und das Speicherelement, die auf das Trägerelement aufgebracht sind, sind mit einem Schutzfilm überdeckt. Dadurch werden diese vor vorzeitigen Beschädigungen geschützt.

Eine weitere alternative Ausgestaltung der Erfassungsvorrichtung sieht vor, dass zumindest die Dehnmesseinrichtung in das Trägermaterial eingewebt ist und vorzugsweise zumindest die Schaltung und/oder das Speicherelement auf einer der beiden Seiten des Trägerelements angeordnet oder in eine Vertiefung des Trägerelementes eingebracht sind. Dadurch kann bereits bei der Herstellung des Trägerelementes eine Integration der Dehnmesseinrichtung ermöglicht sein.

Eine alternative Ausgestaltung der Erfassungsvorrichtung sieht zumindest die Dehnmesseinrichtung und/oder die Schaltung und/oder das Speicherelement und/oder der Beschleunigungssensor zumindest teilweise zwischen dem Trägerelement und einer darauf aufgebrachten Klebeschicht vor. Dadurch können eine einfache Integration und eine noch unmittelbarere Positionierung der Dehnmesseinrichtung zur Haut ermöglicht sein.

Die auf dem Trägerelement aufgebrachte Dehnmesseinrichtung umfasst mehrere Dehnmessstreifen oder mehrere Drahtelemente, der eine Stauchung und/oder Dehnung, die durch den Benutzer bei einer Veränderung seiner Körperhaltung oder eines Bewegungsablaufes auf das Trägerelement überträgt, erfasst. Bevorzugt können zwei oder mehrere Dehnmessstreifen oder Drahtelemente zur Ausbildung der Dehnmesseinrichtung eingesetzt werden. Diese können jeweils separat und räumlich getrennt voneinander auf dem Trägerelement vorgesehen sein und in voneinander abweichende Richtungen weisen. Bevorzugt sind drei übereinander liegende Dehnmessstreifen als Dehnmesseinrichtung vorgesehen, die in Form einer Dehnmessrosette ausgebildet sind, das heißt, dass diese in einer Winkelabweichung von jeweils 45° zueinander ausgerichtet sind. Insbesondere können die übereinander liegenden Dehnmessstreifen durch ein Dielektrikum zueinander getrennt sein. Durch eine Drucktechnologie können die Dehnmessstreifen und das dazwischen angeordnete Dielektrikum nacheinander auf das Trägerelement aufgebracht werden.

Des Weiteren ist bevorzugt vorgesehen, dass der zumindest eine Dehnmessstreifen oder das zumindest eine Drahtelement vorgespannt auf dem Trägerelement vorgesehen ist. Dadurch kann sowohl eine Stauchung als auch eine Dehnung des Trägerelementes durch entsprechende Körperbewegungen wie beispielsweise dem Beugen des Rückens oder der Einnahme einer aufrechten Position gleichermaßen erfasst werden.

Des Weiteren weist die aus mehreren Dehnmessstreifen oder Drahtelemente bestehende Dehnmesseinrichtung bevorzugt zumindest einen Dehnmessstreifen auf, der in das Trägermaterial eingewebt oder aufgenäht ist und/oder zumindest einen Dehnmessstreifen und/oder zumindest ein Drahtelement umfasst, der beziehungsweise das auf dem Trägerelement aufgebracht, insbesondere aufgenäht, eingenäht oder aufgeklebt ist. Diese können dabei gleich oder in verschiedene Richtungen ausgerichtet an dem Trägerelement vorgesehen sein. Das Drahtelement kann als Kett- und/oder Schussfaden eingewebt sein. Das Drahtelement kann auch nachträglich durch Einfädeln in das Gewebe eingebracht sein.

Die Schaltung der Erfassungsvorrichtung kann des Weiteren eine Steuerungselektronik und eine Datenübertragungseinrichtung umfassen. Dadurch können die durch die Dehnmesseinrichtung erfassten und im Speicherelement abgespeicherten Signale ausgelesen werden.

Des Weiteren kann die Erfassungsvorrichtung bevorzugt einen mit der Schaltung verbundenen Stromspeicher umfassen. Dieser kann beispielsweise in Form eines Kondensators, einer Folienbatterie oder einem Lithiumpolymer erfolgen. Dadurch können die Anforderungen an einen in der Höhe geringen Aufbau oder an eine Integration in das Trägerelement erfüllt werden.

Das Trägermaterial der Erfassungsvorrichtung nimmt bevorzugt ein Akustik- und/oder Vibrationselement auf. Dadurch kann bei einer Veränderung der zu überwachenden Körperhaltung oder des zu überwachenden Bewegungsablaufes unmittelbar ein akustisches und/oder vibrierendes Signal ausgegeben werden, welches dem Benutzer umgehend die erfasste Änderung anzeigt, so dass er unmittelbar eine Korrektur vornehmen kann.

Des Weiteren kann gemäß einer weiteren alternativen Ausführungsform der Erfassungsvorrichtung an oder in dem Trägerelement eine Aktivierungstaste vorgesehen sein, durch welche die Schaltung aktivierbar ist. Dadurch kann der Benutzer nach dem Aufbringen der Erfassungsvorrichtung auf der Haut die Erfassung und Überwachung der Körperhaltung an dem Trägerelement selbst starten und gegebenenfalls auch zu einem gewünschten Zeitpunkt wieder beenden.

Alternativ kann die Schaltung unmittelbar mit einem mobilen Endgerät aktivierbar sein. Dabei ist eine kontaktlose Datenübertragung zwischen der Schaltung und dem mobilen Endgerät vorgesehen. Des Weiteren kann das mobile Endgerät alternativ mit der Aktivierungstaste kommunizieren, welche wiederum die Schaltung aktiviert. Bei dieser Ausführungsform kann die Aktivierung der Schaltung sowohl unmittelbar durch die Aktivierungstaste durch den Benutzer oder durch ein mobiles Endgerät erfolgen.

Eine alternative Ausführungsform der Erfassungsvorrichtung sieht vor, dass die Schaltung als passiver oder aktiver Transponder ausgebildet ist und eine Datenübertragungseinrichtung zur kontaktlosen Datenübertragung zwischen einem mobilen Endgerät oder einem Lesegerät und dem Transponder vorgesehen ist. Beispielsweise können die elektrischen Komponenten zu einem passiven Transponder ausgebildet sein, der nach dem Einbringen in ein elektromagnetisches Wechselfeld ein Auslesen der gespeicherten Daten mit einem Lesegerät ermöglicht. Bei der weiteren Ausführungsform als aktiver Transponder wird dem Benutzer über das mobile Endgerät zumindest ein Signal gegeben, dass eine Änderung in der Körperhaltung gegenüber der gewünschten oder optimalen Körperhaltung erfolgt oder eine Abweichung in dem zu überwachenden Bewegungsablauf gegeben ist. Dabei können die Signale in unterschiedlicher Weise auf dem mobilen Endgerät aufbereitet und ausgegeben werden. Es können sowohl akustische Signale, Vibrationen und/oder optische Signale durch das Display des mobilen Endgeräts ausgegeben werden.

Bevorzugt ist auf der Klebeschicht des Trägerelements eine abziehbare Schutzfolie aufgebracht. Dadurch kann die Erfassungsvorrichtung unmittelbar nach dem Abziehen dieser Schutzfolie für eine Benutzung bereitgestellt und in Benutzung genommen werden.

Des Weiteren kann vorgesehen sein, dass eine Erfassungsvorrichtung nach einer der vorgehenden Ausführungsformen drahtlos mit einem mobilen Endgerät und mit einem darauf gespeicherten Datenverarbeitungsprogramm oder mit einem auf ein mobiles Endgerät herunterladbaren Datenverarbeitungsprogramm (App) kommuniziert.

Die Erfindung sowie weitere vorteilhafte Ausführungsformen und Weiterbildungen derselben werden im Folgenden anhand der in den Zeichnungen dargestellten Beispiele näher beschrieben und erläutert. Die Erfindung ist in den Ansprüchen definiert. Es zeigen:
Figur 1 eine schematische Ansicht auf die erfindungsgemäße Erfassungsvorrichtung,
Figur 2 eine schematische Seitenansicht der erfindungsgemäßen Erfassungsvorrichtung gemäß Figur 1,
Figur 3 eine schematische Seitenansicht einer alternativen Ausführungsform der Erfassungsvorrichtung zu Figur 1,
Figur 4 eine schematische Draufsicht auf eine weitere alternative Ausführungsform der Erfassungsvorrichtung zu Figur 1,
Figur 5 eine schematische Ansicht auf eine weitere alternative Ausführungsform der Erfassungsvorrichtung zu Figur 1,
Figur 6 eine schematische Draufsicht auf eine weitere alternative Ausführungsform der Erfassungsvorrichtung zu Figur 1 und
Figur 7 eine schematische Ansicht auf eine Erfassungsvorrichtung gemäß Figur 1 in Kommunikation mit einem mobilen Endgerät.

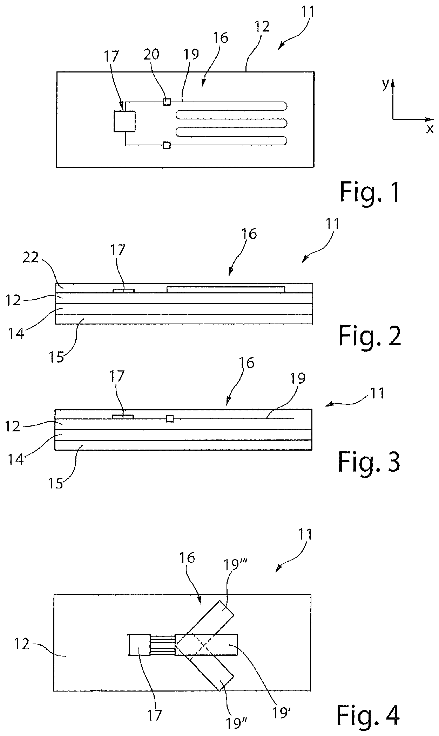

Die Figur 1 zeigt eine schematische Ansicht von oben auf die Erfassungsvorrichtung 11. Eine schematische Seitenansicht hiervon ist in Figur 2 dargestellt. Die Erfassungsvorrichtung 11 besteht aus einem Trägerelement 12, welche auf einer Seite eine Klebeschicht 14 aufweist, um die Erfassungsvorrichtung 11 unmittelbar auf der Haut eines Benutzers zu positionieren. Diese Klebeschicht 14 ist bevorzugt hautverträglich und durch eine Schutzfolie 15 abgedeckt, welche vor dem Aufbringen der Erfassungsvorrichtung 11 auf der Haut des Benutzers abgezogen wird. Alternativ können auf einer Schutzfolie 15 mehrere Erfassungsvorrichtungen 11 nebeneinander angeordnet sein, die einzeln nach Bedarf davon abgenommen werden können.

Auf der der Klebeschicht 14 gegenüberliegenden Seite des Trägerelementes 12 ist eine Dehnmesseinrichtung 16 aufgebracht. Diese ist mit einer Schaltung 17 kontaktiert, welche mit einem Speicherelement verbunden sein kann.

Das Trägerelement 12 ist band- oder streifenförmig ausgebildet. Bevorzugt kann dieses aus einem Gewebe bestehen. Alternativ können atmungsaktive und/oder luftdurchlässige Folien als auch ein Vlies oder Filz vorgesehen sein, welche zumindest in eine Erstreckungsrichtung eine Dehnung und/oder Stauchung zulassen.

Die Dehnmesseinrichtung 16 kann als ein auf einer Folie aufgebrachtes Element auf dem Trägerelement 12 vorgesehen sein. Alternativ kann die Dehnmesseinrichtung 16 auch direkt auf das Trägerelement 12 aufgedruckt werden. Beispielsweise kann dies durch eine leitfähige Paste aufgedruckt, aufgesprüht oder aufgerakelt werden, um den Dehnmessstreifen 19 und die dazugehörigen Kontaktstellen 20, welche die Dehnmesseinrichtung 16 bilden, aufzubringen. Benachbart dazu kann die Schaltung 17, insbesondere ein IC-Chip, aufgebracht werden. Dieser kann wiederum mit einem Speicherelement verbunden sein. Zum Schutz der elektronischen Komponenten ist bevorzugt auf dem Trägerelement 12 eine Schutzschicht 22, insbesondere ein Schutzlack, aufgebracht, der die elektrischen Komponenten vorzugsweise vollständig abdeckt.

Die vorbeschriebene Dehnmesseinrichtung 16 kann alternativ zu dem Dehnmessstreifen 19 auch durch zumindest ein Drahtelement ausgebildet werden, welches wie beispielsweise in Figur 1 dargestellt ist, mäanderförmig oder zick-zack-förmig auf dem Trägerelement 11 aufgebracht, aufgenäht und/oder sogar in das Trägerelement 11 eingewebt ist. Durch solche Drahtelemente kann ebenfalls eine Änderung des Widerstandes bei einer Dehnung oder Stauchung erfasst und ausgewertet werden.

Die Erfassungsvorrichtung 11 ist in einer ersten Erstreckungsrichtung entlang der X-Achse länger als in der zweiten Erstreckungsrichtung, der Y-Achse, ausgebildet. Dieses Trägerelement 12 ist dazu geeignet, um zumindest ein Dehnen und Stauchen entlang der ersten Erstreckungsachse, also der X-Achse, aufzunehmen. Dementsprechend ist die Dehnmesseinrichtung 16 auch zur ersten Erstreckungsrichtung ausgerichtet. Durch eine solche Erfassungsvorrichtung 11 kann beispielsweise eine Änderung in der Körperhaltung erfasst werden, insbesondere eine Änderung einer ergonomisch günstigen Haltung oder Sitzposition. Beispielsweise kann diese Erfassungsvorrichtung 11 entlang einer Wirbelsäule ausgerichtet am Rücken, insbesondere einer Rückenmuskulatur, beispielsweise zwischen den Schulterblättern, aufgebracht werden. Sofern eine zunehmende Krümmung des Rückens erfolgt, indem aufgrund einer Nachlässigkeit oder Müdigkeit die ergonomisch günstige Sitzposition verlassen wird, erfolgt durch die Dehnmesseinrichtung 16 eine Dehnung in X-Richtung, welche in diesem Fall eine Erstreckung längs der Wirbelsäule entspricht. Eine solche Änderung kann erfasst und gegebenenfalls abgespeichert oder angezeigt werden.

In Figur 3 ist eine schematische Seitenansicht einer alternativen Ausführungsform der Erfassungsvorrichtung 11 zu den Figuren 1 und 2 dargestellt. Die elektrischen Komponenten bei dieser Ausführungsform gemäß Figur 3 entsprechen denen der Figuren 1 und 2. Abweichend hiervon ist die Dehnmesseinrichtung 16 in das Trägerelement 12 eingearbeitet oder eingebettet. Beispielsweise kann der Dehnmessstreifen 16 bei der Ausgestaltung des Trägerelementes 12 als ein Gewebe als Widerstandsdraht ausgebildet und unmittelbar in das Gewebe eingewebt sein, ebenso die Kontaktstellen 19 zum Anschluss der Schaltung 17. Die Schaltung 17 und gegebenenfalls das Speicherelement können an einer der beiden Seiten des Trägerelementes 12 angebracht sein oder beispielsweise zumindest teilweise in eine Vertiefung im Trägerelement 12 eingebracht werden. Sofern die Vertiefung zur Klebemittelschicht 14 weisend und gegenüberliegend eine durchgehend geschlossene Oberfläche durch das Trägerelement 12 gebildet ist, kann eine Schutzschicht 22 entfallen.

In Figur 4 ist eine alternative Ausführungsform zu Figur 1 in einer Draufsicht dargestellt. Bei dieser Ausführungsform ist die Dehnmesseinrichtung 16 aus drei übereinander liegenden Dehnmessstreifen 19 als sogenannte Dehnmessrosette ausgebildet. Der sich in der ersten Erstreckungsrichtung erstreckende Dehnmessstreifen 19' wird einem zweiten und einem dritten Dehnmessstreifen 19", 19'" überlagert, die einmal um plus 45° und minus 45° gegenüber der ersten Erstreckungsrichtung ausgerichtet sind. Durch eine solche Dehnmesseinrichtung 16 können Änderungen sowohl in der ersten als auch in der zweiten Erstreckungsrichtung - also in X- und Y-Richtung - und alle dazwischen liegenden in der XY-Ebene liegenden Kraftrichtungen erfasst, ausgewertet und gegebenenfalls angezeigt werden.

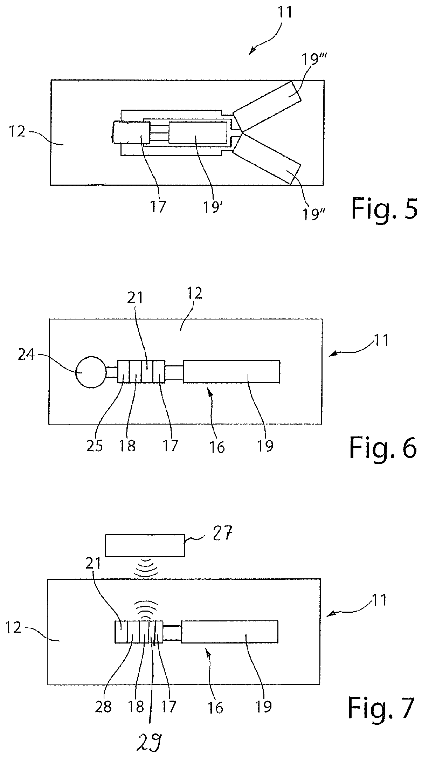

In Figur 5 ist eine alternative Ausführungsform zu Figur 4 dargestellt. Diese weicht dahingehend ab, dass die drei Dehnmessstreifen 19', 19", 19"' nicht übereinander liegend, sondern separat am oder im Trägerelement 12 vorgesehen sind. Hinsichtlich der Funktionsweise entspricht diese Ausführungsform der in Figur 4.

Die vorbeschriebenen Ausführungsformen der drei Dehnmessstreifen 19', 19 " und 19"', welche als vorgefertigte Elemente aufgebracht werden, gilt bezüglich deren Ausrichtung analog für einzelne Drahtelemente, die auf das Trägerelement 12 aufgenäht oder in das Trägerelement 12 eingewebt sind. Bei Verwendung von mehreren Drahtelementen kann beispielsweise eines der Drahtelemente in das Gewebe des Trägermaterials eingewebt und ein weiteres Drahtelement auf das Trägerelement 12 aufgenäht sein.

Figur 6 zeigt eine erste Ausführungsform der Erfassungsvorrichtung 11, welche um einen Stromspeicher 21, eine Aktivierungstaste 24 und ein Vibrationselement 25 und/oder ein akustisches Element ergänzt ist. Diese Erfassungsvorrichtung 11 kann unabhängig von weiteren technischen Hilfsmitteln zur Überwachung einer Körperhaltung oder eines Bewegungsablaufes eingesetzt werden und ein Speicherelement 18 aufweisen.

Durch die Aktivierungstaste 24 wird die Erfassungsvorrichtung 11 nach dem Aufbringen auf der Körperregion oder des Körperteils aktiviert und nach dem Durchlaufen einer Kalibrierroutine in den Überwachungsmodus gesetzt. Bei einer Änderung der Körperhaltung oder des zu überwachenden Bewegungsablaufes kann durch das Vibrationselement 25 und/oder das Akustikelement ein entsprechendes Signal abgegeben werden, welches dem Benutzer die Änderung mitteilt.

Des Weiteren kann die Aktivierungstaste 24 und/oder die Schaltung 17 derart ausgebildet sein, dass die Erfassungseinrichtung 11 auch kontaktlos mittels eines mobilen Endgeräts 27 gestartet werden kann, indem diese die Aktivierungstaste 24 oder die Schaltung 17 aktiviert. Bei dieser Ausführungsform kann die Erfassungsvorrichtung 11 sowohl ohne die Verwendung eines mobilen Endgerätes 27 als auch unter Verwendung des mobilen Endgerätes 27 benutzt werden.

In Figur 7 ist eine alternative Ausführungsform der Erfassungsvorrichtung 11 zu Figur 6 dargestellt. Bei dieser Ausführungsform ist ergänzend zu den Komponenten der Schaltung 17, dem Speicherelement 18, dem Datenübertragungsmodul 28 und/oder der Energiequelle 21 ein Beschleunigungssensor 29 vorgesehen. Durch diesen Beschleunigungssensor 29 kann eine weitere Lage- und Positionserkennung erfasst werden, wodurch die Überwachung präzisiert wird und weitere Überwachungsparameter abgefragt werden können.

Bei dieser Ausführungsform in Figur 7 kommuniziert die Erfassungsvorrichtung 11 mit einem mobilen Endgerät 27. Hierzu ist die Schaltung 17, die auch ein Speicherelement 18 umfasse kann, mit einer Datenübertragungseinrichtung 28 gekoppelt, so dass die durch die Schaltung 17 erfassten Signale kontaktlos an das mobile Endgerät 27 übertragen werden. Ebenso kann über das mobile Endgerät 27 die Erfassungsvorrichtung 11 nach dem Aufbringen auf dem Körperteil oder der Körperregion gestartet werden. Das mobile Endgerät 27 kann ein Smartphone, ein Tablet, eine Uhr oder ein weiteres Lesegerät sein, welches zum kontaktlosen Auslesen von Daten geeignet ist. Hierzu wird auch ein Lesegerät verstanden, welches einen aktiven oder passiven Transponder auslesen und/oder mit diesem kommunizieren kann.

Die vorbeschriebenen Ausführungsformen gemäß den Figuren 1 bis 6 können ebenfalls fakultativ mit einem solchen Beschleunigungssensor 29 ausgebildet sein. Auch können die Ausführungsformen gemäß den Figuren 1 bis 5 ergänzend ein Kontaktspeicherelement 18 und/oder eine Energiequelle 21 und/oder eine Aktivierungstaste 24 und/oder ein Vibrationselement 25 und/oder ein Datenübertragungsmodul 28 und/oder ein Beschleunigungssensor 29 umfassen.

Des Weiteren kann vorgesehen sein, dass bei einer mittigen Anordnung und Aufbringung der Dehnmesseinrichtung 16 die weiteren elektronischen Komponenten wie beispielsweise die Schaltung 17, das Speicherelement 18, die Energiequelle 21, die Aktivierungstaste 24, das Vibrationselement 25, das Datenübertragungsmodul 28 und/oder der Beschleunigungssensor 29 außermittig aufgebracht werden, so dass eine Beeinträchtigung bei der Dehnung und/oder Stauchung des Trägermaterials durch diese Komponenten in der Achse der Dehnmesseinrichtung 16 nicht gegeben ist.

Bei dem Verwenden einer neuen Erfassungsvorrichtung kann die Kopplung oder das Binden der Erfassungsvorrichtung an das mobile Endgerät durch einen Code erfolgen. Dieser Code kann auf dem Trägermaterial der Erfassungsvorrichtung und/oder auf einer Verpackung der Erfassungsvorrichtung vorgesehen sein. Dabei kann es sich bei einem solchen Code beispielsweise um einen Barcode, QR-Code, Nummern-Code oder dergleichen handeln.

## Patentansprüche

1. Erfassungsvorrichtung (11) zum Erfassen und Überwachen einer Körperhaltung oder eines Bewegungsablaufs oder eines Trainingsablaufs eines Körperteils mit einer Dehnmesseinrichtung (16), welche ein Trägerelement (12) aufweist, das tapeförmig ausgebildet ist und auf einer Seite eine Klebeschicht (14) zum Aufkleben auf der Haut eines Benutzers aufweist, wobei die Dehnmesseinrichtung (16) an oder in dem Trägermaterial (12) angeordnet ist, welche Messsignale in zumindest einer Erstreckungsrichtung des Trägerelements (12) durch eine Dehnung und/oder Stauchung des Trägerelementes (12) erfasst, und die Dehnmesseinrichtung (16) mit einer als Transponder ausgebildeten Schaltung (17) kontaktiert und an dem Trägerelement (12) befestigt ist, wobei die Dehnmesseinrichtung (16), die Schaltung (17), ein Speicherelement (18) für die durch die Dehnmesseinrichtung erfassten Signale und ein Beschleunigungssensor (29) auf dem Trägermaterial (12) aufgebracht und mit einer Schutzschicht (22) überdeckt sind, die auf dem Trägermaterial (12) aufgebracht ist, und das Trägerelement (12) mit der Dehnmesseinrichtung (16) und der Schaltung (17) als Einwegprodukt ausgebildet ist.

2. Erfassungsvorrichtung nach Anspruch 1, **dadurch gekennzeichnet, dass** das Trägerelement (12) aus einem Gewebe, Gewebeband, einer atmungsaktiven und/oder luftdurchlässigen Folie, aus einem Vlies oder einem Filz hergestellt ist.

3. Erfassungsvorrichtung nach einem der vorhergehenden Ansprüche, **dadurch gekennzeichnet, dass** zumindest die Dehnmesseinrichtung (16) oder die Schaltung (17) oder das Speicherelement (18) und/oder der Beschleunigungssensor (29) auf das Trägerelement (12) aufgeklebt, aufgedruckt, aufgegossen, aufgerakelt oder aufgesprüht sind.

4. Erfassungsvorrichtung nach einem der Ansprüche 1 oder 3, **dadurch gekennzeichnet, dass** zumindest die Dehnmesseinrichtung (16), die Schaltung (17), das Speicherelement (18) oder der Beschleunigungssensor (29) zumindest teilweise zwischen dem Trägerelement (12) und der Klebeschicht (14) vorgesehen ist.

5. Erfassungsvorrichtung nach einem der Ansprüche 1 bis 3, **dadurch gekennzeichnet, dass** zumindest die Dehnmesseinrichtung (16) in das Trägermaterial (12) eingewebt oder aufgenäht ist und zumindest die Schaltung (17), das Speicherelement (18) oder der Beschleunigungssensor (29) auf einer der beiden Seiten des Trägermaterials (12) angeordnet oder zumindest teilweise in einer Vertiefung des Trägermaterials (12) eingebracht sind.

6. Erfassungsvorrichtung nach einem der vorhergehenden Ansprüche, **dadurch gekennzeichnet, dass** die Dehnmesseinrichtung (16) zwei oder drei Dehnmessstreifen (19) oder Drahtelemente mit Kontaktstellen (20) umfasst, die jeweils separat und räumlich getrennt zueinander oder in Form einer Dehnmessrosette ausgebildet sind, die vorzugsweise über ein Dielektrikum zueinander getrennt und übereinander liegend aufgebracht sind.

7. Erfassungsvorrichtung nach Anspruch 6, **dadurch gekennzeichnet, dass** zumindest ein Dehnmessstreifen (19) oder zumindest ein Drahtelement in das Trägermaterial (12) eingewebt ist oder zumindest ein Dehnmessstreifen (19) oder zumindest ein Drahtelement auf dem Trägerelement (12) aufgebracht ist.

8. Erfassungsvorrichtung nach einem der vorhergehenden Ansprüche, **dadurch gekennzeichnet, dass** die Schaltung (17) eine Steuerelektronik zur Auswertung der Messsignale oder eine Datenübertragungseinrichtung oder beides umfasst.

9. Erfassungsvorrichtung nach einem der vorhergehenden Ansprüche, **dadurch gekennzeichnet, dass** die Schaltung (17) mit einem Stromspeicher (21) kontaktiert ist.

10. Erfassungsvorrichtung nach einem der vorhergehenden Ansprüche, **dadurch gekennzeichnet, dass** das Trägermaterial (12) ein Akustikelement oder Vibrationselement (25) oder beides aufweist.

11. Erfassungsvorrichtung nach einem der vorhergehenden Ansprüche, **dadurch gekennzeichnet, dass** das Trägermaterial (12) eine Aktivierungstaste (24) zum Aktivieren der Schaltung (17) aufweist oder dass die Schaltung (17) unmittelbar mit einem mobilen Endgerät (27) aktivierbar ist oder dass die Schaltung (17) über die Aktivierungstaste (24) durch das mobile Endgerät (27) aktivierbar ist.

12. Erfassungsvorrichtung nach einem der vorhergehenden Ansprüche, **dadurch gekennzeichnet, dass** die Schaltung (17) und das Speicherelement (18) als ein passiver oder aktiver Transponder ausgebildet sind und eine kontaktlose Datenübertragung zwischen einem mobilen Endgerät (27) oder einem Lesegerät und dem Transponder vorgesehen ist.

13. Erfassungsvorrichtung nach einem der vorhergehenden Ansprüche, **dadurch gekennzeichnet, dass** auf der Klebeschicht (14) eine abziehbare Schutzfolie vorgesehen ist.

14. Erfassungsvorrichtung nach Anspruch 8, **dadurch gekennzeichnet, dass** die Datenübertragungseinrichtung eingerichtet ist, drahtlos mit einem auf einem mobilen Endgerät (37) gespeicherten Datenverarbeitungsprogamm oder einem herunterladbaren Datenverarbeitungsprogamm zu kommunizieren.

## Claims

1. A detecting device (11) for detecting and monitoring a body posture or a movement sequence or a training sequence of a body part with a strain measuring device (16), which comprises a carrier element (12) which is formed in the shape of a tape and has an adhesive layer (14) on one side for adhering to the skin of a user, wherein the strain measuring device (16) is arranged on or in the carrier element (12), which detects measuring signals in at least one direction of extension of the carrier element (12) by an extension and/or compression of the carrier element (12) and the strain measuring device (16) is contacted with a circuit (17) designed as a transponder and is fastened to the carrier element (12), wherein the strain measuring device (16), the circuit (17), a storage element (18) for the signals detected by the strain measuring device and an acceleration sensor (29) being applied to the carrier material (12) and covered with a protective layer (22) applied to the carrier material (12), and the carrier element (12) with the strain measuring device (16) and the circuit (17) being designed as a disposable product.

2. Detection device according to claim 1, **characterized in that** the carrier element (12) is made of a fabric, fabric tape, a breathable and/or air-permeable film, a nonwoven or a felt.

3. Detection device according to one of the preceding claims, **characterized in that** at least the strain measuring device (16) or the circuit (17) or the memory element (18) and/or the acceleration sensor (29) are glued, printed, cast on, squeegeed on or sprayed onto the carrier element (12).

4. Detection device according to one of claims 1 or 3, **characterized in that** at least the strain measuring device (16), the circuit (17), the memory element (18) or the acceleration sensor (29) is provided at least partially between the carrier element (12) and the adhesive layer (14).

5. Detection device according to any one of claims 1 to 3, **characterized in that** at least the strain measuring device (16) is woven or sewn into the carrier material (12) and at least the circuit (17), the memory element (18) or the acceleration sensor (29) are arranged on one of the two sides of the carrier material (12) or are at least partially introduced into a recess of the carrier material (12).

6. Detection device according to one of the preceding claims, **characterized in that** the strain measuring device (16) comprises two or three strain gauges (19) or wire elements with contact points (20), which are each formed separately and spatially separated from one another or in the form of a strain gauge rosette, which are preferably separated from one another via a dielectric and are applied one above the other.

7. Detection device according to claim 6, **characterized in that** at least one strain gauge (19) or at least one wire element is woven into the carrier material (12) or at least one strain gauge (19) or at least one wire element is applied to the carrier element (12).

8. Detection device according to one of the preceding claims, **characterized in that** the circuit (17) comprises control electronics for evaluating the measurement signals or a data transmission device or both.

9. Detection device according to one of the preceding claims, **characterized in that** the circuit (17) is contacted with a current storage device (21).

10. Detection device according to any one of the preceding claims, **characterized in that** the carrier material (12) comprises an acoustic element or vibration element (25) or both.

11. Detection device according to one of the preceding claims, **characterized in that** the carrier material (12) comprises an activation button (24) for activating the circuit (17) or that the circuit (17) is activateable directly by a mobile terminal (27) or that the circuit (17) is activateable by the mobile terminal (27) via the activation button (24).

12. Detection device according to one of the preceding claims, **characterized in that** the circuit (17) and the memory element (18) are designed as a passive or active transponder and contactless data transmission is provided between a mobile terminal (27) or a reader and the transponder.

13. Detection device according to any one of the preceding claims, **characterized in that** a peelable protective film is provided on the adhesive layer (14).

14. Acquisition device according to claim 8, **characterized in that** the data transmission device is arranged to communicate wirelessly with a data processing program stored on a mobile terminal (37) or a downloadable data processing program.

## Revendications

1. Dispositif de détection (11) destiné à détecter et à surveiller une posture corporelle ou une séquence de mouvements ou une séquence d'entraînement d'une partie du corps grâce à un dispositif extensométrique (16) qui présente un élément support (12) réalisé en forme de ruban et présentant, sur une face, une couche adhésive (14) destinée à être collée sur la peau d'un utilisateur, dans lequel le dispositif extensométrique (16) est disposé sur ou dans le matériau support (12) et détecte des signaux de mesure dans au moins une direction d'extension de l'élément support (12) provoqués par une élongation et/ou un aplatissement de l'élément support (12) et le dispositif extensométrique (16) est connecté à un circuit (17) réalisé en tant que transpondeur et est fixé à l'élément support (12), dans lequel le dispositif extensométrique (16), le circuit (17), un élément de stockage (18) destiné à stocker les signaux détectés par le dispositif extensométrique et un capteur d'accélération (29) sont appliqués sur le matériau support (12) et sont recouverts par une couche de protection (22) qui est appliquée sur ledit matériau support (12), et l'élément support (12) pourvu du dispositif extensométrique (16) et du circuit (17) est réalisé en tant que produit à usage unique.

2. Dispositif de détection selon la revendication 1, **caractérisé en ce que** l'élément support (12) est réalisé en un tissu, une bande de tissu, un film respirant et/ou perméable à l'air, en un non-tissé ou un feutre.

3. Dispositif de détection selon l'une quelconque des revendications précédentes, **caractérisé en ce qu'**au moins le dispositif extensométrique (16) ou le circuit (17) ou l'élément de stockage (18) et/ou le capteur d'accélération (29) sont appliqués sur l'élément support (12) par collage, par impression, par coulage, à la racle ou par vaporisation.

4. Dispositif de détection selon l'une quelconque des revendications 1 ou 3, **caractérisé en ce qu'**au moins le dispositif extensométrique (16), le circuit (17), l'élément de stockage (18) ou le capteur d'accélération (29) est prévu au moins en partie entre l'élément support (12) et la couche adhésive (14) .

5. Dispositif de détection selon l'une quelconque des revendications 1 à 3, **caractérisé en ce qu'**au moins le dispositif extensométrique (16) est tissé dans le matériau support (12) ou est cousu sur celui-ci et qu'au moins le circuit (17), l'élément de stockage (18) ou le capteur d'accélération (29) sont disposés sur l'une des deux faces du matériau support (12) ou sont insérés au moins en partie dans un creux du matériau support (12).

6. Dispositif de détection selon l'une quelconque des revendications précédentes, **caractérisé en ce que** le dispositif extensométrique (16) comprend deux ou trois rubans extensométriques (19) ou éléments en fil métallique pourvus de points de contact (20) qui sont réalisés respectivement de manière individuelle et séparée spatialement les uns des autres ou sous la forme d'une rosette extensométrique et qui de préférence sont séparés les uns des autres par un diélectrique et sont appliqués de manière superposée.

7. Dispositif de détection selon la revendication 6, **caractérisé en ce qu'**au moins un ruban extensométrique (19) ou au moins un élément en fil métallique est tissé dans le matériau support (12) ou **en ce qu'**au moins un ruban extensométrique (19) ou au moins un élément en fil métallique est appliqué sur l'élément support (12).

8. Dispositif de détection selon l'une quelconque des revendications précédentes, **caractérisé en ce que** le circuit (17) comprend une électronique de commande destinée à évaluer les signaux de mesure ou un dispositif de transmission de données ou les deux à la fois.

9. Dispositif de détection selon l'une quelconque des revendications précédentes, **caractérisé en ce que** le circuit (17) est connecté à un accumulateur de courant (21).

10. Dispositif de détection selon l'une quelconque des revendications précédentes, **caractérisé en ce que** le matériau support (12) présente un élément acoustique ou un élément vibrant (25) ou les deux à la fois.

11. Dispositif de détection selon l'une quelconque des revendications précédentes, **caractérisé en ce que** le matériau support (12) présente une touche d'activation (24) destinée à activer le circuit (17) ou **en ce que** le circuit (17) peut être activé de manière directe grâce à un terminal mobile (27) ou que le circuit (17) peut être activé grâce au terminal mobile (27) par l'intermédiaire de la touche d'activation (24).

12. Dispositif de détection selon l'une quelconque des revendications précédentes, **caractérisé en ce que** le circuit (17) et l'élément de stockage (18) sont réalisés sous la forme d'un transpondeur passif ou actif et qu'une transmission de données sans fil est prévue entre un terminal mobile (27) ou un appareil de lecture et ledit transpondeur.

13. Dispositif de détection selon l'une quelconque des revendications précédentes, **caractérisé en ce qu'**un film de protection pelliculable est prévu sur la couche adhésive (14).

14. Dispositif de détection selon la revendication 8, **caractérisé en ce que** le dispositif de transmission de données est conçu pour communiquer sans fil avec un programme de traitement de données stocké sur un terminal mobile (37) ou avec un programme de traitement de données téléchargeable.
